# EUROPEAN PATENT APPLICATION

(11) **EP 1 566 640 A1**
(43) Date of publication of application: **24.08.2005**
(21) Application number: 04003603.0
(22) Date of filing: 18.02.2004
(51) Int. Cl.: G01N 33/558, G01N 33/543, B01L 3/00

(54) **Sampling device, the method and use thereof**

(71) Applicant: Ani Biotech Oy, 00100 Helsinki (FI)
(72) Inventor: Niskanen, Aimo, 02660 Espoo (FI); Saramäki, Mika, 03620 Karkkila (FI)
(74) Representative: Lönnqvist, Gunnel Solveig Kristina

(57) **Abstract**

The invention provides an immunochemical sampling device and use thereof, said sampling device comprising an elongated support surrounded at one of its proximal ends by a porous layer. The porous layer is optionally surrounded by an impermeable protecting layer and optionally also attached to the elongated support at the same time by means of the protecting layer. The porous layer comprises a specific labeled binding reagent which is activated by a liquid sample and mobilized in a controlled manner when contacted with an analyzer device comprising a porous carrier. The porous carrier of the analyzer device comprises at least one specific binding reagent in a detection zone. The analyzer device can be used with a multiple channel analyzer device in order to detect several analytes from one sample. The result can be read from the detection zone of the analyzer device directly visually or by appropriate equipment capable of recording the results. Additionally the invention provides an immunochemical method for determining the presence of absence of an analyte in a liquid sample and a process for collecting a sample for immunochemical testing.

## Description

### FIELD OF THE INVENTION

The present invention relates to an immunochemical sampling device, especially to devices which are suitable for home use, at doctors officies and/or by technically untrained staff, involving a minimal level of skills from the users.

### BACKGROUND OF THE INVENTION

Methods and devices based on immunodiffusion are known from for example US 4,757,002, US 3,990,852 and US 4,562,147. Immunochromatographic methods based on lateral flow are known from EP 0 291 194, EP 0 284 232, EP 0 250 137, US 5,250,412 US 5,084,245, US 6,375,896 and WO 86/03839.

US 4,562,147 provides a radial immunodiffusion enzyme assay method for testing of pseudorabies antibodies in swine and other animals. Agar test plates are provided including an underlying adherent coating of solubilized non-infectious swine pseudorabies antigen. The result of the test is obtained from the diameters of the resulting colored zones which correlate with the titers obtained by the official virus neutralization test.

EP 0 291 194 relates to assays involving specific binding, especially immunoassays and devices therefor. The analytical test device comprises a hollow casing, containing a dry porous carrier, which communicates indirectly with the exterior of the casing via a bibulous sample receiving member. The carrier contains in a first zone a labeled specific binding reagent and in a second zone spatially distinct from the first zone an unlabelled specific binding reagent for the same analyte. A disadvantage of this method compared to the present invention is that the sample solution is contacted with the test device when the test is performed, which increases the risk of overflow to the second zone.

EP 0 284 232 provides a solid phase assay for determining the presence or absence of analyte in a liquid sample. A test strip of the invention has a tracer movably supported on a first portion and a binder immobilized on a second portion. A disadvantage of this method is that the sample solution is contacted with the test strip when the test is performed, which increases the risk of overflow to the second portion.

EP 0 250 137 describes an immunoassay using colloidal gold for detecting a ligand in a sample, where a membrane strip is contacted with a sample and simultaneously or successively with a liquid reagent containing a ligand binding partner or ligand labeled with colloidal gold. The disadvantage of this method compared with the present invention is the need of an additional liquid reagent containing the labeled ligand.

US 5,250,412 describes a swab device, method and kit for collecting and analyzing an analyte. The sample is collected with the swab of the invention, but a separate liquid containing a labeled component has to be applied to the swab and subsequently also a wash solution. The labeled component reacts immunochemically with the sample and the immunoassay is carried out within the device. The disadvantages of this method, i.e. the separate liquid containing a labeled component and the separate wash solution can be avoided by the present invention where all parts can be dried and hermetically packed.

US 5,084,245 provides a device for conducting diagnostic procedures based on immunological reactions using specimens gathered up in the absorbent tip of a swab. When used the swab is pushed through a passageway towards a sensitive element. Ribs are positioned in the passageway to squeeze the tip and express fluid of the swab. The results are visually observable by removing a guide member from the base component to uncover the sensitive element.

US 6,375,896 describes a swab analyzer for the immunochemical detection of substances. A swabbing pen of the swab analyzer is used for the collecting of the sample. The sample is however eluted with an elution liquid, which is a clear disadvantage compared to the present invention.

WO 86/03839 illustrates a solid phase diffusion assay where the sample is first mixed with a labeled binding substance and then applied to a region of a support with immobilised adsorbent molecules and allowed to diffuse therein. The diffusion pattern is visualized and measured. The present invention provides several advantages to this assay. First separate liquids are avoided, thus enabling smaller packages and tests suitable for home use. Secondly more than one analyte can be tested at the same time, with the same small amount of sample.
It is evident from the description of the background art that a multitude of different test kits are available. A wide variety of test kits are available commercially and many of them are intended for home use. In spite of their convenient formats, there are many risks for errors if they are used in an erroneous manner. This risk is imminent when the test device is used for sampling, which may be both impractical and inconvenient. Especially, when the test device is used as a sampling device or for collecting the sample there is a risk that the sensitive reagents and the structure of the analytical device is destroyed or disturbed. The present invention provides an improved detection system for immunochemical tests involving a sampling device which enables the liquid sample to be collected and the labeled specific binding reagent to be transferred and mobilized in a controlled manner. Due to the two-part system the analyzer device is not in direct contact with the liquid sample, thereby minimizing the possibilities that the reagents in and the structure of the analyzer device are destroyed or disturbed by the sampling procedure.

### SUMMARY OF THE INVENTION

The present invention provides an immunochemical sampling device comprising an elongated support surrounded at one of its proximal ends by a porous layer, optionally with an impermeable protecting layer wherein said porous layer comprises a labeled specific binding reagent which is activated by the liquid sample and mobilized in a controlled manner when the sampling device has been contacted with a analyzer device (result device) comprising a porous carrier.

The invention provides a sampling device for collecting a sample for lateral flow testing. The sampling device comprises an elongated support (core stick). A porous layer is applied around one end of the core stick. The porous layer comprises a labeled specific binding reagent and has been treated with a blocking solution preventing reactive groups of the porous material to react with the sample or the labeled specific binding reagent. The sampling device may be contacted with the liquid to be tested and subsequently brought in contact with a porous carrier of the analyzer or result device comprising at least one specific binding reagent immobilized as a dot or zone.

The immunochemical sampling device enables a controlled mobilization of the labeled specific binding reagent in the analyzer device comprising a porous carrier. Thereby a more sensitive method for detecting different analytes in liquid samples is provided. The analytes to be detected can be disease specific antibodies including IgG, IgM and IgA, antibodies against *Helicobacter pylori,* Hepatitis A, HIV_{1,2}, respiratory disorders or etc., antigens excreted to the urine including Luteinizing hormone (LH), Follicle stimulating hormone (FSH) and human chorionic gonadotropin (hCG) or for example antigens of or antibodies against bacteria, virus, fungi and parasites or components and products thereof. The device and method of the invention may be used for a multitude of different tests including pregnancy, menopause, fertility, Thyroid stimulating hormone, toxoplasmosis, cancer antigen, respiratory disorder, allergy, myocardial infarct and drug tests, etc.

The elongated support (core stick) of the sampling device is an elongated stick made of wood or plastic, for example polypropylene (PP) and polyvinylchloride (PVC) surrounded at its proximal ends by a porous layer and an impermeable layer comprising one or more, preferably 1-5 layers of tape for adjusting the flow of the labeled binding reagent from the sampling device to the analyzer device. The porous layer of the sampling device also comprises one or more layers, preferably 1-5 layers of a porous material. The porous material is selected from a group of materials consisting of paper, glass fiber, nylon, polyester or cellulose and derivatives thereof.

The porous layers of the sampling device comprise at least one specific labeled binding reagent, which is impregnated either to part or to the whole of the porous material of the sampling device.

The porous carrier of the analyzer device comprises at least one specific binding reagent directly or indirectly immobilized as a dot or zone (test line). Moreover one or more of the dots or zones on the porous carrier may act as a control zones. It is obvious for one skilled in the art that the sampling device can be used in connection with a analyzer device where the porous carrier comprises one porous passage, which may be penetrated by a sample solution, containing detection zone(s) but also with a analyzer device where the porous carrier comprises two or more channels optionally made by a suitable method comprising at least one specific binding reagent per channel, immobilized as a dot or zone. The porous material of the analyzer device is selected from a group of materials consisting of nitrocellulose, paper, glass fiber, nylon, polyester, polysulphonate or cellulose and derivatives thereof.
Different types of analyzer devices are described in background art. Devices with multiple channels enable testing of several analytes simultaneously. Markers specific for different analytes can be grouped together to form different products. The multiple channel device comprises a porous carrier processed by a water-repellency treatment or otherwise in order to cause a network of channels where the tested sample can migrate. Different specific binding reagents may be bound in each channel.

The analyzer device of the present invention differs from the conventional prior art devices in that it lacks the mobilizable specific reagent.

The specific reagents of the sampling device and the analyzer device include antibodies, antibody fragments, recombinant antibodies, recombinant antibody fragments, antigens, lectins, receptors and/or ligands. The label applicable in the sampling device includes colored latex, gold, metal, dye, fluorogenic substances, superpara-magnetic particles coated with the specific binders. Chromogenic substances, particularly fluorochromogens and enzymatic labels may be used as markers as well.

The blocking solution for making the porous material inert is a mixture comprising natural or synthetic polymers such as albumin (BSA, Bovine serum albumin) and casein or PEG (polyethylene glycol), PVA (polyvinyl alcohol) and PVP (polyvinyl pyrrolidone), nonionic detergents such as HEXA (hexane sulphonic acid) and TRITON-X-100, SDS, BRIJ and preservation agents such as sugar, for example glucose, sucrose and trehalose or derivatives thereof.

The device is dried to a moisture content of 8 % or less and packed hermetically separately or in combination with said analyzer device (result recording detecting device).

The detection of an analyte in a liquid sample is detected by bringing the sampling device into contact with the liquid sample and further with the analyzer device. The liquid sample alongside with the labeled specific binding reagent is allowed to migrate from the diagnostic sampling device to the porous carrier of the result device from which the positive or negative results are directly readable. The result can be read directly visually (by naked eye) or by appropriate equipment capable of recording the results.
The invention further provides a detection system comprising an immunochemical sampling device and an analyzer device, where the sampling device is contacted or left in contact with the analyzer device and the liquid sample and the labeled specific binding reagent or the reaction product (complex) formed thereof are allowed to migrate or flow from the sampling device to the porous carrier of the analyzer device comprising a specific binding reagent optionally immobilized on the porous carrier. Normally the liquid from the sampling device moves throughout the porous carrier of the analyzer device by diffusion and/or capillary action.

The liquid sample can be urine, blood, serum, plasma, saliva or a sample buffer solution. In a particularly preferred embodiment the liquid sample is urine.

Besides the increased security of the test results other advantages of the sampling device and detection system of the invention are; the small format of the sampling device and the analyzer device which leads to material savings, less waste products and decreased freight costs and thus environmentally friendlier products. Further as the test system is easy to use it enables home use. Due to the fact that the analyzer device is not in direct contact with the liquid sample, overflow is avoided and an increased reliability of the test is obtained. Moreover the sampling device and analyzer device of the invention are easy to store due to the fact that the devices are dried and that they are possible to store hermetically.

### BRIEF DESCRIPTION OF THE DRAWINGS

FIG. 1 is a perspective view of a sampling device in accordance with the invention;
FIG. 2 is a view seen from down under of the sampling device;
FIG. 3 is a view seen from down under of another embodiment of the sampling device;
FIG. 4 is a side view of the sampling device and the analyzer device;
FIG. 5 is a view seen from above of the analyzer device.
FIG. 6 is a view seen from above of another embodiment of the analyzer device.

### DETAILED DESCRIPTION OF THE INVENTION

This invention provides an immunochemical sampling device comprising an elongated support surrounded at one of its proximal ends by a porous layer, optionally with an impermeable protecting layer wherein the porous layer comprises a specific labeled binding reagent (immunoreagent) which is activated by a liquid sample and mobilized in a controlled manner when contacted with an analyzer device comprising a porous carrier.

Also a method for determining the presence or absence of an analyte in a liquid sample is provided in the invention. The method comprises the steps of bringing the diagnostic sampling device in contact with the liquid sample, bringing the diagnostic sampling device in contact with the analyzer device, allowing sufficient time for the liquid sample alongside with the labeled specific binding reagent or the reaction product thereof to migrate or flow from the diagnostic sampling device to the porous carrier of the analyzer device where the reaction product of the liquid sample and the labeled binding reagent moves towards the detection zone of the analyzer device and reading the result by detecting the presence or absence of the analyte in the detection zone.

In one embodiment of the invention the analyzer device comprises both a detection zone and a control zone. More detection zones can be added in order to perform a quantitative test.

In another preferred embodiment of the invention a multiple channel analyzer device is used in order to detect the same sample for several analytes at the same time. Every channel of this device can thereto have several detection zones and/or control zones.

### Constructing the sampling device

The construction of the sampling device comprises the steps of; treating a porous material with a blocking solution, drying, adding a labeled specific binding reagent to the porous material, drying, attaching the porous material around an elongated support in order to form a porous layer(s), optionally attaching an impermeable material layer around the porous layer and finally drying and packing the device. It is also possible to first attach the porous material around the support and to thereafter immerse or otherwise apply a blocking solution and a labeled specific binding reagent to the porous layers of the sampling device.

### Making the porous material inert

A blocking solution, a mixture comprising natural or synthetic polymers such as albumin (BSA, Bovine serum albumin) and casein or PEG (polyethylene glycol), PVA (polyvinyl alcohol) and PVP (polyvinyl pyrrolidone), nonionic detergents such as TWEEN 20, HEXA (hexane sulphonic acid), TRITON-X-100, SDS and BRIJ and preservation agents such as sugar, for example glucose, sucrose and trehalose or derivatives thereof, is added to the porous layer in order to make the reactive sites of the porous material inert. The blocking solution can be added by a pump or the porous material can be immersed in the solution.

Before the next step the porous material is dried.

### Adding the specific labeled reagent

The porous layer of the sampling device is impregnated with a specific labeled reagent. The whole layer can be impregnated but preferably the labeled specific binding reagent is impregnated at the lower end of the layer. The labeled specific binding reagent is obtained by coating the label particles with specific binding reagent(s) using methods well known in the art. The labeled specific binding reagent may be applied using tube pumps, which deliver precise volumes of the reagent through a needle or alternatively the porous material can be immersed. It is possible to use a combination of several different labeled specific binding reagents if the sample is detected for more than one analyte and the same specific binding reagent cannot be used for all.

Antibodies, antibody fragments, recombinant antibodies, recombinant antibody fragments, antigens, lectins, receptors and/or ligands are suitable reagents, which can be attached to colored latex, gold, metal, dye, fluoroscent substances or superpara-magnetic particles. It is however possible to use chromogenic substances, particularly fluorochromogens and enzymatic labels as markers as well. The porous material is dried in a dry room, with a relative humidity less than 20 % and further in a dry room with a relative humidity less than 8%.

### Attaching the materials

One or more layers of the porous material are attached around an elongated support made of for example wood or plastic. One or more layers can be attached one at a time or a strip of the porous material can be rolled around the support stick. The porous material can be attached by a tape which is first attached to the elongated support, then rolled with the porous material around the stick and finally the tape is attached to itself. The support stick can be an elongated round, flat or planar stick, which is solid or hollow. Optionally one or more layers of an impermeable material such as tape are attached around the porous layer(s).

### Drying and packing the device

The device is dried to a moisture content of 8 % or less and packed hermetically separately or in combination with said analyzer device.

### Preparing the analyzer device

The analyzer device, which is the result recording detecting device of the detection system, can be prepared by immobilizing one or more specific binding reagents and optionally also control reagents directly or indirectly to the porous carrier of the device before blocking. The blocking solution used can be the same as the one used for the sampling device. The porous carrier can optionally be placed on a backing or in a casing. A multiple channel device can be prepared by treating a porous material with a suitable method in order to get different channels for tests of different analytes (or controls). Several analytes can be detected from the same sample by one single test.

### Use of the detection system

The sampling device can be used by holding the device under the urine stream or by immersing the sampling device in a liquid sample or adding a sample by pipette. If the sampling device is put under the urine stream the preferred time is 2-15 seconds, preferably 5-15 seconds and most preferably 5-10 seconds. If the sampling device is immersed in a liquid sample, the preferred time is 2-30 seconds, preferably 10-20 seconds, most preferably 10-12 seconds. If the liquid sample is added by pipette the preferred amount of sample is 2-40 drops, preferably 5-20 drops and most preferably 5-15 drops. Ten drops is equivalent to about 0,5 ml.

### The structure of the sampling device and the analyzer device

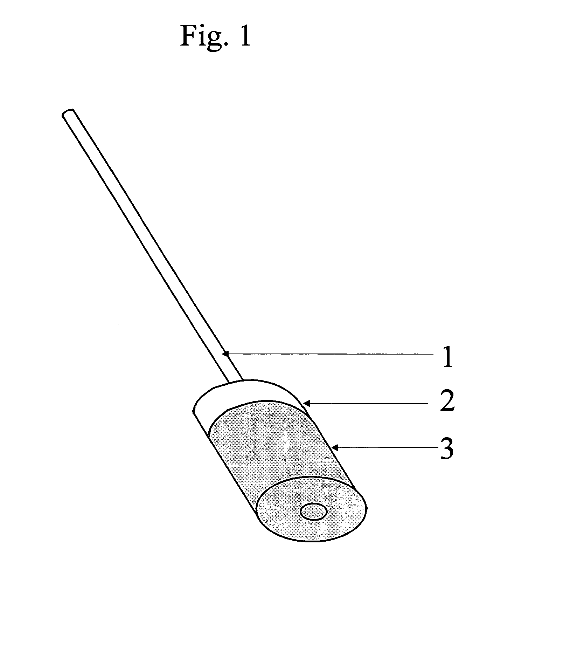

FIG. 1 represents a typical sampling device. An elongated hollow support 1 is surrounded by porous layers of a porous material 2 at one of its proximal ends. The porous material comprises a labeled specific binding reagent 3 at the lower end of the porous material.

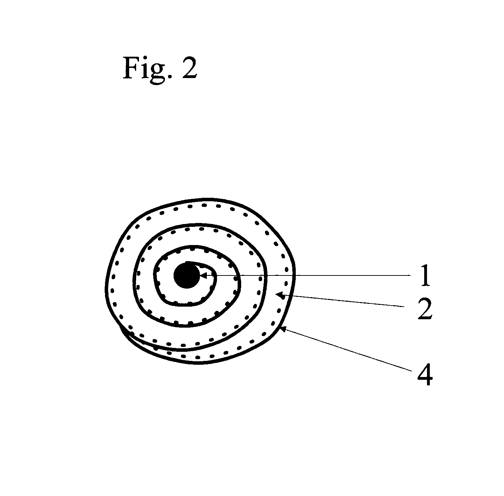

FIG. 2 shows the sampling device from down under. Four layers of a porous material 2 is rolled around the solid support 1. The porous layer is protected by an impermeable protecting layer 4.

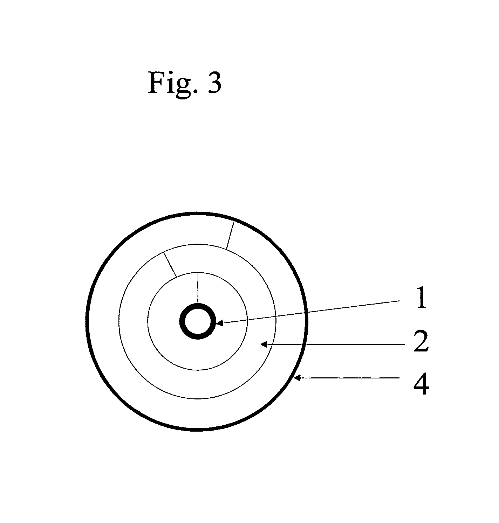

FIG. 3 shows another embodiment of the view seen from down under of the sampling device. The hollow support 1 is surrounded by three layers of a porous material 2 and the porous material is surrounded by one or more layers of an impermeable protecting layer 4.

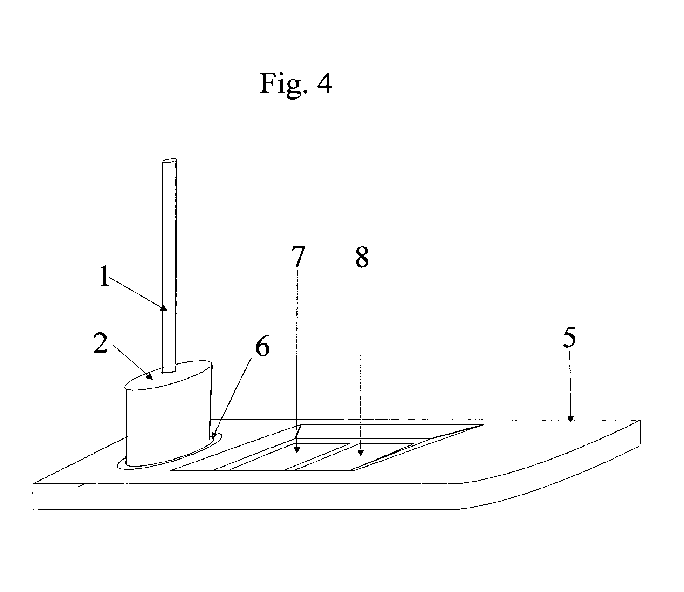

FIG. 4 represents a typical detecting system of the invention. The sampling device comprising a support 1 surrounded by a porous material 2 is contacted with the analyzing device comprising a casing 5 with a sample well 6, a test window 7 and a control window 8.

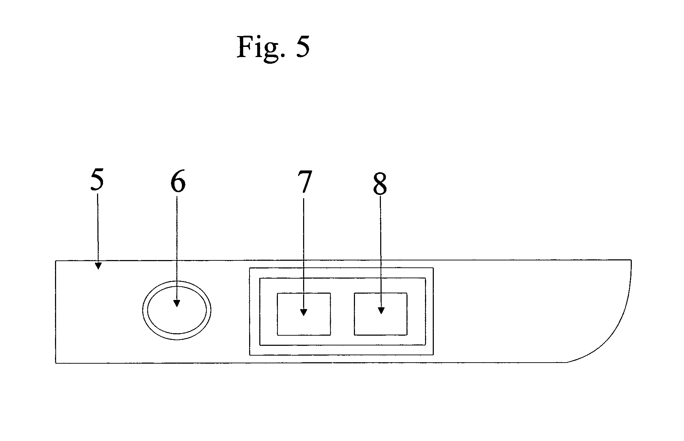

FIG. 5 shows a typical analyzer device seen from above. A casing 5 comprises a sample well 6 where the sampling device is placed when it has been contacted with the liquid sample. The detection zone of the porous carrier is placed in the test window 7 and in case of a positive result a visible reaction can be seen there. The control zone of the porous carrier is placed in the control window 8 where a visible reaction should be seen whenever the test is performed.

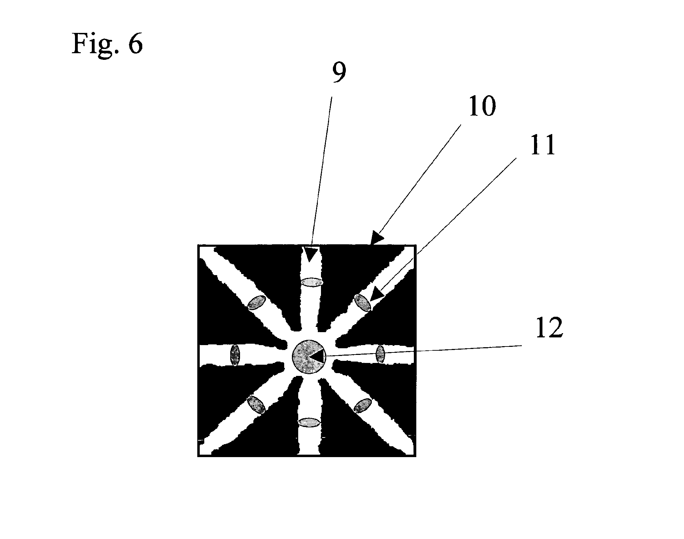

FIG. 6 shows another embodiment of an analyzer device. Eight channels 9 are made by a suitable method on a porous carrier. The channels of the device are separated from each other by treated areas 10. Each channel 9 comprises a detection zone or dot 11. Also a place 12 (sample or sampling device receiver) where the sampling device should be contacted is shown.

The embodiments described in the Figures and the Examples are only to be seen as examples of embodiments which are within the scope of the invention. They should not be considered to limit the scope of the invention as defined by the claims.

### EXAMPLE 1 Pregnancy test

Example 1 relates to a hCG pregnancy test, in which a protecting impermeable layer is added to the sampling device in order to attach and protect the porous layer containing the specific labeled reagent. A control zone is added to ensure a proper performance of the test.

The sampling device of Fig. 1 was constructed by using a porous polyester filter material as a platform for the first reaction between the gold conjugate and the hCG antigen. The polyester material obtainable as rolls measuring 100 meters in length and 10 mm in width was used.

The polyester material was first blocked with a blocking solution comprising BSA (0,1-1,0 %), Tween 20 (0,01-0,05 %) and trehalose (0,5-1,5 %). After drying, 3 µl/mm of a gold conjugate solution which comprised a hCG specific binding reagent was applied to the blocked polyester. The solution was added to a 5 mm wide area at the end of the material using tube pumps and was then left to dry in a dry room, with a relative humidity less than 20%. The drying was continued in a dry room with a humidity of less than 8 %.

The polyester filter was cut in 10 mm x 30 mm pieces. Four layers of the porous material was rolled around a round hollow stick made of polypropylene and attached with a tape. The tape was first attached to the polypropylene stick and then rolled with the polyester around the stick and finally the tape was attached to itself. The edge with the impregnated labeled specific binding reagent was set in the lower end of the sampling device.

The analyzer device of Fig. 5 was constructed by immobilizing a hCG specific antibody on the porous carrier to form a detection zone. A monoclonal antibody against the labeled specific binding reagent was immobilized on the carrier to form a control zone. The nitrocellulose was then blocked with a blocking solution comprising BSA (0,1-5,0%), TRITON-X-100, BRIJ and saccharose. The material was allowed to dry. The porous carrier was placed in a casing.

The sampling device and the analyzer device was dried to 8 % moisture content and packed hermetically in a hermetic pouch.

The end containing the porous layer of the sampling stick was placed under a urine stream for 10 seconds. Thereafter the sampling stick was put in the sample well of the analyzer device. The complex formed of the sample and labeled specific binding reagent was allowed to migrate for 5 minutes after which the result was read in the test window. Red lines were visible in the detection zone of the test window and in the control zone of the control window, which indicated the presence of hCG in the sample.

### EXAMPLE 2 Pregnancy test

Example 2 relates to a hCG-pregnancy test where the porous layer containing the specific labeled reagent is attached without a impermeable layer. A control zone is added to ensure a proper performance of the test.

A polyester filter was pretreated as described in Example 1. One layer of the filter was adhered with an adhesive around a solid stick of wood. The analyzer device was made as in Example 1 and finally the devices were dried and packed as described in Example 1.

The test was performed by immersing the sampling device for 10 seconds in a liquid sample containing hCG. The stick was shaken lightly and thereafter placed in the sample well of the analyzer device. The presence of hCG was detected as described in Example 1.

### EXAMPLE 3 Fertility test detection system

Example 3 describes how the invention can be applied for a fertility test detection system.

A cellulose filter was blocked with a blocking solution comprising BSA (0,1-1,0 %), Tween 20 (0,01-0,05%) and trehalose (0,5-1,5%).

After drying, 3 µl/mm of a gold conjugate solution which comprised three labeled specific antibodies for hCG, LH and FSH was applied to the blocked material. The solution was added to a 3 mm wide area at one edge of the material using tube pumps and was then left to dry in a dry room, with a relative humidity less than 20%.

The cellulose filter was cut in 10 mm x 20 mm pieces. One piece of the cellulose filter was rolled as four layers around a round hollow stick made of polypropylene and attached with a tape. The edge with the impregnated binding reagent was set in the lower end of the sampling device.

The analyzer device of Fig. 5 was constructed by blocking a porous carrier of nitrocellulose with a blocking solution comprising BSA (0,1-1,0 %), Tween 20 (0,01-0,05%) and trehalose (0,5-1,5%). The material was allowed to dry before immobilizing 1 mg/ml of hCG, LH and FSH specific antibodies on the porous carrier to form three different detection zones. 1 mg/ml of a monoclonal antibody against the labeled specific binding reagent was immobilized on the carrier to form a control zone. The porous carrier was dried and placed in a casing.

The test was performed by immersing the end containing the porous layer of the sampling stick in a liquid sample of urine for 10 seconds. The sampling stick was then put in the sample well of the analyzer device. The complex formed of the sample and labeled specific binding reagent was allowed to migrate for 5 minutes after which the result was read in the test window. Red lines were visible in the detection zone of the test window and in the control zone of the control window, which indicated the presence of either all or one or more of hCG, LH or FSH in the sample.

### EXAMPLE 4 Detection system for venereal diseases

Example 4 relates to a detection system for HIV_{1,2}.

A polyester filter, used as the porous material of the sampling device was blocked with a blocking solution comprising BSA (0,1-5,0%), TRITON-X-100, BRIJ and saccharose. After drying, 2 µl/cm of a colored latex solution which comprised a polypeptide recognizing both HIV₁ and HIV₂ was applied to the blocked material. The solution was added to 4 mm of the filter and the filter was then left to dry in a dry room, with a relative humidity less than 20%.

The polyester filter was cut in 10 mm x 25 mm pieces. One piece of the polyester filter was rolled three times around a round hollow stick made of wood and attached with tape.

The analyzer device with two test windows and one control window was constructed by immobilizing 0,5 µl of a HIV₁ recombinant antigen and 0,5 µl of a HIV₂ recombinant antigen on the porous carrier to form the detection zones on a porous carrier of nitrocellulose. The material was allowed to dry before the nitrocellulose was blocked with a blocking solution comprising BSA (0,1-5,0%), TRITON-X-100, BRIJ and saccharose. A monoclonal antibody against the labeled specific binding reagent was immobilized on the porous carrier to form a control zone. The porous carrier was dried and placed in a casing. The control zone was placed farthest away from the sample well of the analyzer device.

The test was performed by immersing the end containing the porous layer of the sampling stick in a sample of serum. The sampling stick was put in the sample well of the analyzer device. The sample and labeled specific binding reagent was allowed to migrate for 5 minutes after which the result was read in the test windows. The result was read after 10 and 15 minutes as well. As a result red lines were visible in the detection zones of the test window and in the control zone of the control window, which indicated the presence of HIV_{1,2} in the sample.

### EXAMPLE 5 Multiple channel test for allergens

Example 5 describes use of the invention for allergic testing. The same sample is at the same time tested for several allergens using a multiple channel analyzer device.

A polyester filter, used as the porous material of the sampling device was blocked with a blocking solution comprising BSA (0,1-5,0%), TRITON-X-100, BRIJ and saccharose. After drying, 10 µl of a water solution of colored latex particles coated with anti IgE antibodies recognizing specific IgE molecules was applied to the blocked material. The solution was added to the whole filter and was then left to dry in a dry room, with a relative humidity less than 20%.

The polyester filter was cut in10 mm x 20 mm pieces. One piece of the polyester filter was rolled four times around a round hollow stick made of polypropylene attached with tape.

The analyzer device with eight separated channels was constructed by forming the channels in a porous carrier of nitrocellulose and a mylard film 0,5 µl of eight different specific allergens were immobilized on the porous carrier to form the detection zones in each channel of the analyzer device. The material was allowed to dry before the nitrocellulose was blocked with a blocking solution comprising BSA, HEXA and trehalose. The porous carrier was dried.

The test was performed by immersing the end containing the porous layer of the sampling stick in a liquid sample of serum. The sampling stick was contacted with the place of the analyzer device were the sample should be placed. The sample and the labeled specific binding reagent was allowed to migrate for 5 minutes after which the result was read as visible dots in the detection zones, which indicated the presence of allergens in the sample.

## Claims

1. An immunochemical sampling device for performing an immunoassay comprising an elongated support surrounded at one of its proximal ends by a porous layer **characterized in that** said porous layer comprises a labeled specific binding reagent which is activated by a liquid sample and mobilized in a controlled manner when contacted with a porous carrier of an analyzer device.

2. The immunochemical sampling device according to claim 1 **characterized in that** said porous layer is surrounded by an impermeable protecting layer.

3. The immunochemical sampling device according to claim 2 **characterized in that** said protecting layer comprises one or more layers of tape.

4. The immunochemical sampling device according to any of the preceding claims **characterized in that** said porous layer comprises one or more layers of a porous material.

5. The immunochemical sampling device according to any of the preceding claims **characterized in that** the porous layer selected from the group consisting of paper, glass fiber, nylon, polyester or cellulose and derivatives thereof.

6. The immunochemical sampling device according to any of the preceding claims **characterized in that** said porous material of the sampling device is blocked with a blocking solution comprising natural or synthetic polymers, nonionic detergents or preservation agents.

7. The immunochemical sampling device according to any of the preceding claims **characterized in that** said specific binding reagents are selected from the group consisting of antibodies, antigens, lectins, receptors, ligands, fragments thereof or combinations thereof.

8. The immunochemical sampling device according to any of the preceding claims **characterized in that** the label is selected from comprisin colored latex, gold, metal, dye, fluorogenic substances, superparamagnetic substances, chromogenic substances, fluorochromogens or enzymatic labels.

9. The immunochemical sampling device according to any of the preceding claims **characterized in that** said sampling device forms a detection system in combination with said analyzer device.

10. The immunochemical sampling device according to any of the preceding claims **characterized in that** said device is dried to a moisture content of 8 % or less and packed hermetically separately or in combination with said analyzer device.

11. The immunochemical sampling device according to any of the preceding claims wherein said sampling device is contacted with the porous carrier of said analyzer device **characterized in that** said porous carrier lacks a labeled specific binding reagent.

12. Use of the immunochemical sampling device according to any of the preceding claims for detecting pregnancy, menopause, fertility, thyroid stimulating hormone, toxoplasmosis, cancer, respiratory disorder, allergy, myocardial infarct and drugs in urine, blood, serum, plasma, saliva or a sample buffer solution.

13. An immunochemical method for determining the presence or absence of an analyte in a liquid sample comprising the steps of
a. adding the liquid sample to a sampling device according to any one of the preceding claims
b. contacting the sampling device with the analyzer device
c. allowing sufficient time for the complex of the sample and a specific labeled reagent to migrate from the sampling device to the porous carrier of the analyzer device
d. reading the result by detecting the presence or absence of said analyte in the analyzer device.

14. A process for collecting a sample for immunochemical testing, the process comprising the steps of
a. blocking a porous material by a blocking solution
b. applying a labeled specific reagent to the porous material
c. providing an immunochemical sampling device having an elongated support surrounded by said porous material
d. contacting the sampling device with the liquid to be tested
e. contacting the sampling device with a porous carrier of an analyzer device comprising at least one specific binding reagent immobilized as a dot or zone.
